(19) Europäisches Patentamt / European Patent Office / Office européen des brevets

(11) **EP 1 808 125 A1**

(12) **EUROPEAN PATENT APPLICATION**

(43) Date of publication:
**18.07.2007 Bulletin 2007/29**

(51) Int Cl.:
*A61B 5/0452* (2006.01)    *G06F 17/00* (2006.01)
*G06F 19/00* (2006.01)

(21) Application number: **06000737.4**

(22) Date of filing: **13.01.2006**

(84) Designated Contracting States:
**AT BE BG CH CY CZ DE DK EE ES FI FR GB GR HU IE IS IT LI LT LU LV MC NL PL PT RO SE SI SK TR**
Designated Extension States:
**AL BA HR MK YU**

(71) Applicant: **SIEMENS AKTIENGESELLSCHAFT**
**80333 München (DE)**

(72) Inventors:
• **Danehorn, Kenneth**
  **18594 Vaxholm (SE)**
• **Harmat, Peter**
  **22477 Lund (SE)**
• **Milton, Patrik**
  **21152 Malmö (SE)**
• **Pang, Luping**
  **91058 Erlangen (DE)**
• **Stridh, Martin**
  **24565 Hjärup (SE)**
• **Sörnmo, Leif, Prof.**
  **22465 Lund (SE)**

(54) **Electrophysiological system for analysing an intracardiac electrocardiogram**

(57)    The present invention relates to a method for analysing an intracardiac electrocardiogram (IECG), in order to identify at least one of the A wave, V wave and H wave on at least one of the electrogram signals, comprising the steps of pre-processing (14) the electrogram signal; calculating an adaptive threshold for the A, V or H wave, wherein the adaptive threshold depends on the noise level of the electrogram signal and on the type of wave; and identifying the A, V or H wave by searching the electrogram signal within a time window determined e.g. from the position of another wave on the same or another electrogram signal.

FIG 3

EP 1 808 125 A1

**Description**

The present invention relates to a method for analysing an intracardiac electrocardiogram (IECG) consisting of one or several electrogram signals each acquired by a catheter placed inside the heart, in order to identify at least one of the A wave, V wave and H wave on at least one of the electrogram signals. The invention also relates to a electrophysiological system adapted for performing the method, and a computer program product allowing the method to be performed by a computer.

Electrophysiological studies (EPS) are used for diagnosing defects in the heart's conduction system. EPS is carried out by inserting catheters into e.g. the femoral, subclavian, internal jugular or antecubital veins, so they can reach places inside the heart near the sinus node, atrioventricular (AV) node, bundle of His or the ventricles. The catheters can record electrogram signals as well as apply electrical stimulation pulses to stimulate a specific region of the heart. Different diagnostic programs of EPS are defined according to the stimulation pattern and the evaluation.

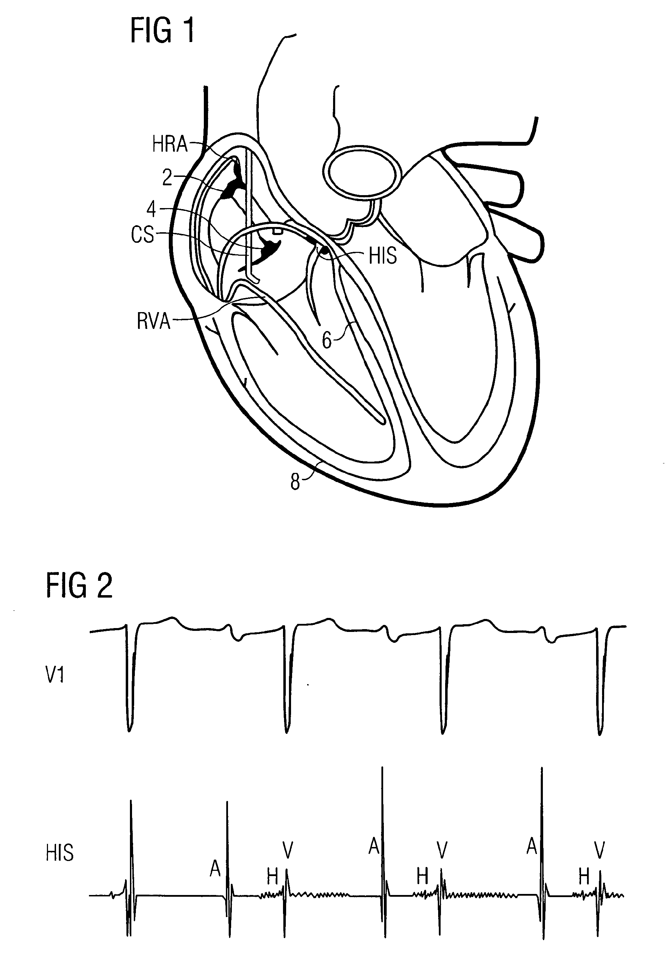

A typical distribution of catheters inside a human heart is illustrated in fig. 1. Fig. 1 shows a four chamber view of the heart with particular focus on the electrical conduction system. Important components of the electrical conduction system of the heart include the sinus node 2, the AV node 4, the bundle of His 6 and the Purkinje fibres 8. The electric activity of a heart beat generally starts at the sinus node 2, which rhythmically initiates 70 - 80 impulses per minute without any nerve stimulation. Depolarisation then propagates through the atrial myocardium and causes the two atria to contract simultaneously. When the impulses reach the AV node 4, they are conducted more slowly. Thereafter, the electrical discharge travels rapidly to the bundle of His, which conducts the impulses to both ventricles, causing ventricular contraction.

The figure further shows a typical set of catheter positions consisting of a high right atrial catheter HRA, a coronary sinus catheter CS, a right ventricular apex catheter RVA and a His bundle catheter HIS.

The signals recorded by these catheters generally show a ventricular potential or V wave, as well as the atrial potential or A wave. The His bundle potential or H wave is generally recorded mainly by the His bundle catheter.

For the diagnosis of heart defects causing arrhythmias, it is generally desirable to be able to measure the time intervals between the different cardiac potentials, such as the A-H interval or the H-V interval.

During an IECG, an ordinary body surface electrocardiogram may also be acquired. Such a BSECG lead V1 recording is shown on the top of fig. 2. The bottom signal of fig. 2 is a His bundle electrogram (HIS) with the AV potential (A), His bundle potential (H) and ventricular potential (V) identified.

It is an object of the invention to provide a method which can automatically identify all visible cardiac activations (A, H or V waves) on an intracardiac electrocardiogram.

This object is achieved by the method according to claim 1, as well as by the electrophysiological system of claim 10 and the computer program product according to claim 11.

The method also uses a simultaneously acquired body surface electrocardiogram (BSECG) and comprises the following steps: Pre-processing the electrogram signal; identifying the QRS-complex in the body surface electrocardiogram; calculating an adaptive threshold for the A, V or H wave, wherein the adaptive threshold depends on the noise level of the electrogram signal and on the type of wave; identifying the A, V or H wave by searching the electrogram signal within a time window determined either from the position of the QRS complex in the BSECG, or the previously determined position of a wave on the same or another electrogram signal, and detecting the A, V or H wave within the time window using the adaptive threshold.

The BSECG is recorded as known in the art. For example, it may be a 12-lead BSECG providing the signals I, II, III, aVL, aVS, aVR and V1 to V6. Procedures for identifying the QRS complex on such BSECG is also known in the art and will not be further described. It is also possible, and may be used in an embodiment of the invention, to detect the P wave in the BSECG.

The number of electrogram signals within the IECG depends on the diagnostic protocol. Since the automatic wave detection of the present invention especially adapted to the His bundle signal, a HIS signal will generally be present. The CS, HRA and RVA or further signals may be available according to the placement of the catheters.

The criteria to identify the A, V or H waves are based on the sequence of the waves appearing under different conditions and their amplitude relationship. Therefore, empirical searching time windows together with adaptive thresholds are used.

Adaptive thresholds mean that the thresholds are set according to the noise level within the electrogram signal. The time windows may be determined either from the position of the QRS complex in the BSECG, or from the position of a wave which has been previously identified on the same or a different electrogram signal.

According to a preferred embodiment, the BSECG is also used to define an R-R interval between two subsequent R waves.

Thereby, it is possible to divide the electrogram signals into R-R intervals each corresponding to one heart beat.

According to another aspect of the invention, the classifycation method is divided into different procedural branches, depending on the position of the catheters, the number of stimulation pulses (if any) and the type of the stimulation. The advantage of such branch definition is that the method can be better adapted to the various applications in an EPS and achieve more accurate results. In order to select the procedural branch, the number of stimulation pulses within the R-R interval is checked. Preferably, this is done on a stimulation marker signal, which is supplied by the EPS system generating the stimulation pulses. In the Siemens cardiac system "SENSIS", this signal is called the "Stim" signal. According to the number of stimulation pulses and the type of the stimulation (antegrade, retrograde), one of several procedural branches may be followed.

According to a preferred embodiment, the pre-processing comprises: applying a high-pass or band stop filter on the electrogram signal, applying a non-linear transformation on the filtered signal in order to extract an envelope of the filtered signal, and applying a low-pass filter on the envelope of the filtered signal. Thereby, it is possible to obtain a positive-valued, smooth signal suitable for further analysis.

In case that stimulation pulses are applied to the heart during the acquisition of the IECG, the method may provide for removal of possible pacing artefacts. Preferably, this is achieved by detecting the ascending or descending edge of a stimulation wave on a stimulation marker signal, such as the "Stim" signal, and setting the electrogram signal to zero within a pre-determined time window around the detected ascending or descending edge.

Preferably, the analysis method is applied on an electrogram signal acquired by a catheter placed near the bundle of His (the HIS signal), and optionally at least one of several further electrogram signals are acquired by catheters placed in the right atrium (the HRA signal), in the coronary sinus (the CS signal), or near the right ventricular apex (the RVA signal).

An example procedural branch for identifying the A wave, H wave and V wave in the HIS signal and the A wave and V wave on the HRA or CS signal is defined in claim 7. In case only a HIS signal needs to be analysed, this procedural branch may be reduced to the steps relating to the HIS signal. This procedural branch is preferably used when no stimulation pulses are used. Other procedural branches having, for example, different adaptive thresholds and different empirical windows, may be used under other conditions, for example during antegrade or retrograde stimulation. Examples for such different procedural branches shall be given below.

According to a further preferred embodiment, the adaptive thresholds are calculated after pre-processing of the signals. However, additional thresholds values may also be calculated from the signals before pre-processing and used to confirm that a certain threshold is reached.

The invention is further directed to an electrophysiological system comprising a data analysis station, which is adapted for performing the above method, and to a computer program product containing program code which, when installed on a computer, will allow the computer to perform the above method.

Examples and preferred embodiments of the invention shall now be described in detail with reference to the accompanying drawings, in which

Fig. 1     is a cross-sectional view of a human heart;

Fig. 2     shows an exemplary body surface ECG recorded on lead V1, and an intracardiac ECG recorded with the HIS bundle electrode;

Fig. 3     is a flow diagram illustrating the main steps of an analysis method according to an embodiment of the invention;

Fig. 4     is a flow diagram illustrating the steps of pre-processing;

Fig. 5     is a flow diagram illustrating the step of the non-linear transform of fig. 4 in more detail;

Fig. 6     illustrates the effects of pre-processing on a HIS signal, where A is the raw signal, B is the high-pass filtered signal, C is the filtered signal after non-linear transformation and D is the low-pass filtered signal of C;

Fig. 7     is a flow diagram showing the steps of an embodiment of a procedural branch;

Fig. 8     shows a body surface ECG and the intracardiac ECG consisting of HIS, HRA and RVA signals.

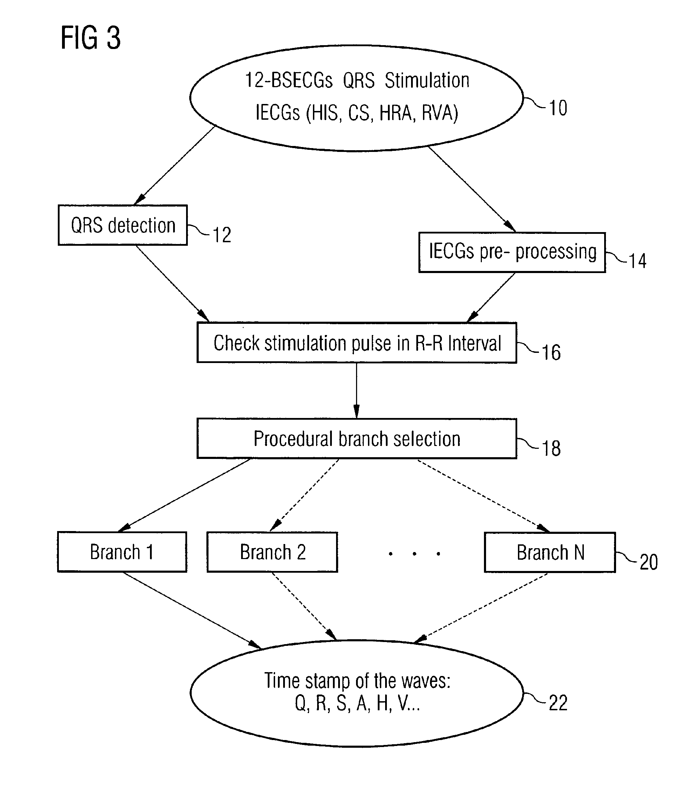

Fig. 3 gives an overview of a method according to an embodiment of the invention. Box 10 contains the input signals from the EPS system, for example the Siemens "SENSIS" system. In this example, this is a 12-lead body surface ECG, as well as an intracardiac ECG containing HIS, CS, HRA and RVA electrogram signals. In addition, the EPS system also furnishes a stimulation marker signal containing information about any external stimulation to the heart, such as the "Stim" signal.

The body surface ECG is then used for QRS detection by means of any available method 12. A pre-processing is applied to the IECG signals in step 14.

The QRS detection is used to divide each intracardiac electrogram signal into R-R intervals. In step 16, one or several selected R-R intervals are checked for stimulation pulses. This information is used to select one of several procedural branches 20 in step 18, but may also be used to determine whether a pacing artefact removal must be performed.

Hence, for the actual classification of the A, H and V waves, the method automatically divides into one of N procedural branches, depending on the presence and number of stimulation pulses, and on the type of available signals. After one of the algorithm branches is completed, the detected waves are provided with a time stamp indicating their type, i.e. A, H, V etc. and the results are outputted to the EPS system in step 22.

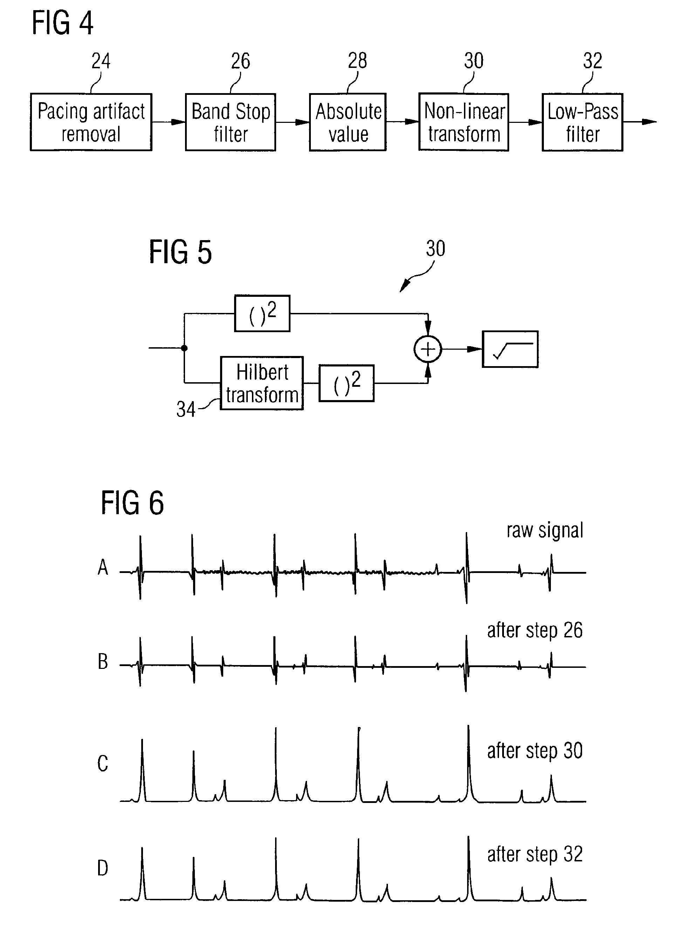

Some of the steps illustrated in fig. 3 shall be described in greater detail in the following. Starting with the pre-processing step 14, reference is made to fig. 4 showing the pre-processing in greater detail. According to fig. 4, the pacing artefact removal 24 is performed before filtering of the signal, though in other embodiments it may be performed after the other pre-processing steps.

If a stimulation pulse is sent to one electrode of a catheter, e.g. the HRA 12 catheter, this may cause distortion of the other electrogram signals, which is called "pacing artefact". To remove such artefact, a stimulation marker signal from the EPS system is used, which indicates each stimulation pulse as a short, preferably square stimulation wave. This stimulation wave may be detected for example by its ascending edge. Due to the nature of the stimulation marker signal, there is a 10 ms time delay between the beginning of a pacing artefact and the ascending edge of the stimulation wave. Besides, it is supposed that the width of the pacing artefact is not larger than 20 - 30 ms. Therefore, a 10 - 30 ms, preferably 20 ms window is selected around the ascending edge and the effected IECG signals (HIS, HRA, RVA or CS) are set to zero within this window.

The first step 26 of the pre-processor is a linear, time invariant band stop filter, designed to suppress ringing noise in the base line, see signal A in fig. 6. The band stop filter ideally suppresses all frequencies between about 30 and 70 Hz. The cut-off frequencies should be selected without loss of any important clinical information, and without significant effect on the wave form. Instead of the band stop filter, a high-pass filter with a cut-off frequency of around 70 Hz may also be used.

In step 28, the absolute value of the filtered signal is taken. Next, a non-linear transformation is performed in step 30. This step is designed to calculate a deterministic, positive-valued signal y(n), referred to as the envelope of the filtered signal x(n).

The details of step 30 are illustrated in fig. 5. A linear, time-invariant filter known as the Hilbert transform is used. The effect of the steps in fig. 5 may be written as

$$y(n) = |x(n)| + 2/\pi |x(n+1) - x(n-1)|$$

Due to the calculated difference between x(n+1) and x(n-1), the Hilbert transform amplifies high gradients and thereby produces easily detectable peaks.

Finally, a low-pass filter with a cut-off frequency of approximately 40 Hz is applied in step 32. The result of this filter is shown as signal D in fig. 6.

For performing the wave identification in one of the N branches, the electrogram signals are first sub-divided into R-R intervals by means of the BSECG. Whenever a new QRS complex is detected in the BSECG signal (V1 or another lead), the time window between the last two QRS complexes (R-R interval) will be focussed on to perform the wave identification in the IECG signals and their envelope signals.

In order to define the procedural branch, the number of the stimulation pulses within the R-R interval is checked in the "Stim" signal. According to the number of stimulation pulses and the type of stimulation (antegrade, retrograde), the algorithm will for example go to one of the following N=5 branches to identify the A, V and H wave in the IECG. Of course, further branches may be added, or some of the branches omitted, according to the individual requirements.

Branch 1 -     No stimulation pulse in the R-R interval, one catheter is placed in high right atrial or coronary sinus to get the HRA or CS signal.

Branch 2 -     One antegrade stimulation pulse in the R-R interval, the stimulation is placed either in the HRA or CS signal.

Branch 3 -     Two antegrade stimulation pulses in the R-R interval, the stimulation is placed either in the HRA or CS signal.

Branch 4 -     One retrograde stimulation pulse in the R-R interval, the stimulation is placed either in the RVA signal.

Branch 5 - No stimulation pulse in the R-R interval, only the HIS signal is acquired; no catheter is placed in high right atrial or coronary sinus.

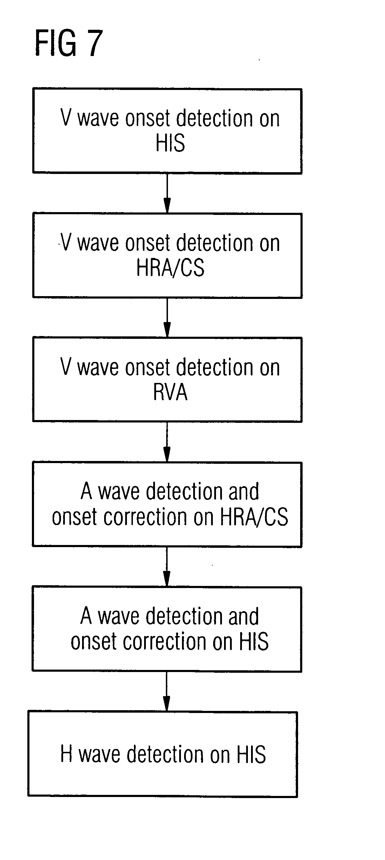

The main steps of branch 1 are illustrated in fig. 7. The other branches may be modifications of this branch. According to fig. 7, the V wave onset is first detected on the HIS signal, then on the HRA or CS signal and finally on the RVA signal. Then the A wave is searched in the CS/HRA, and then the HIS signal; H wave detection in the HIS signal is the last step.

Each detection step uses a windowing technique, where the position of the window is determined based on the results of a previous detection step and the width of the window is generally pre-determined and empirically developed.

Within these windows, the filtered signal is searched to find either the time point where the signal crosses a certain adaptive threshold, which is defined as the onset of a wave, or the window is searched for a maximum, which must also be above a certain threshold.

Adaptive thresholds mean that the thresholds are set according to the estimation of the average noise level (baseline) in the filtered IECG signals. IECG signals have a time-variant signal to noise ratio, so that the average noise level should be calculated for each R-R interval. In a preferred embodiment, the R-R interval is first divided into 8 sub-segments; then the local maximums are calculated for each sub-segment. The minimal local maximum value is regarded as the baseline or noise level in this signal segment.

The adaptive thresholds for the different wave detections are written as:

$$\text{Thr\_}\Phi\text{\_X} = \alpha * \text{Baseline\_X}$$

where X is the IECG signal (such as HIS, HRA, CS or RVA) or the respective envelope signal, $\Phi$ is the type of wave to be detected (A, V or H) and $\alpha$ is a pre-determined value, preferably an integer value, which is empirically determined.

In the following, examples are given for 5 different procedural classification branches, which are chosen according to the above list.

According to requirements, either a threshold on the preprocessed envelope signal, or on the raw signal, may be used in the different procedural branches.

In the following examples, the indication V_HIS indicates the detected position of the V wave onset on the HIS signal, A_CS indicates the A wave onset in the CS signal, etc.

Branch 1

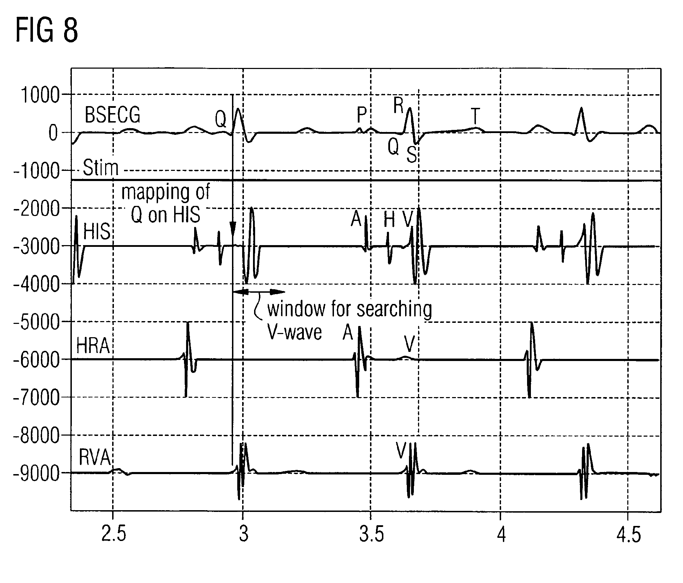

The source signals of branch 1 are the BSECG II, HIS, and HRA or CS signals. No stimulation pulse exists in the current R-R interval (Fig. 8).

1. V wave onset detection in HIS (V_HIS): To define the time window, the position of the Q wave is taken or "mapped" from the BSECG onto the HIS signal. If the mapping point on the HIS signal is over the V wave threshold Thr_V_HIS, the onset of the V wave is backward searched in the HIS signal within a 30ms window. Otherwise, the onset of the V wave is forward searched in the filtered HIS signal until the signal amplitude is over the threshold Thr_V_HIS.

2. V wave onset detection in HRA or CS (V_HRA/CS): The detected V wave onset on HIS signal is mapped to the HRA or CS signal. The V wave maximum in the HRA or CS is searched in a 100ms window after the mapping point. Then, the V wave onset in HRA or CS is backward corrected in a 30 ms window using the threshold Thr_V_HRA/CS.

3. V wave onset detection in RVA (V_RVA): The V wave maximum in the RVA is searched within the 100ms around the mapping point from the R wave in the BSECG. Its onset is also backward corrected in a 30 ms window using the threshold Thr_V_RVA.

4. A wave detection in the HRA or CS (A_HRA/CS): In the HRA or CS, the A wave has larger potential so that it can be more easily detected. So the A wave is first detected in the HRA or CS signal. The maximum value is searched between the first V wave + 80 ms to the second V wave onset in the R-R interval. If this maximum is over the threshold Thr_A_HRA/CS, it is detected as A wave in the HRA or CS. Thr_A_HRA is larger then Thr_A_CS because the A wave potential is mostly higher in the HRA signal than in the CS signal. Once the A wave is confirmed in the HRA or CS signal, its onset is corrected by a threshold of 1/10 of the A wave maximum in the signal. If no A wave is found in the HRA or CS signal, no A wave is searched in the HIS signal.

5. A wave detection in the HIS (A_HIS): The window [A_HRA/CS - 50ms : A_HRA/CS + 60ms] is applied to the HIS signal. The maximum within this window is detected as the A wave in the HIS signal. The onset correction of the A wave consists of two steps: first, it is backward corrected by the threshold Thr_A_HIS in a 20 ms window in the HIS signal; second, a forward correction is done on the filtered HIS signal by the threshold Thr_A_HIS .

6. Multi-A waves detection: A multi-A waves detection is additionally considered for specific arrhythmia conditions (e.g., atrial flutter). If the first A wave is found in the R-R interval, other local peaks will be further searched in the two windows: [A_HRA/CS + 100ms : V_HRA/CS - 80ms] and [Last_V_HRA/CS + 100ms: A_HRA/CS - 80ms]. If the local peak is over 60% of the first A wave amplitude in the HRA or CS signal, it is detected as another A wave. Then the corresponding multi-A waves are further searched in the HIS signal.

7. H wave detection in the HIS (H_HIS): The window for searching the H wave in the HIS signal depends on the position of A and V wave and their distance. If no A wave is detected, H wave is searched in a fixed window [V_HIS - 60ms : V_HIS - 15ms]. If A wave is detected, the beginning of the window is adjusted according to the distance between the V wave onset and the A wave, that is, whether the AV interval is larger than 150ms. In the window, the local peak that is over the threshold Thr_H_HIS is detected as H wave. If there is no local peak, the maximal value that is over the same threshold is considered as H wave.

Branch 2

In this branch, one antegrade stimulation pulse is placed in the R-R interval, which indicates the existence of the signal HRA or CS.

1. V wave onset detection in HIS: V wave onset detection has the same process as that in branch 1.

2. V wave onset detection in HRA or CS: The detected V wave onset on HIS signal is mapped to the HRA or CS signal. The V wave maximum in the HRA or CS is searched in a 60 ms window after the mapping point.

3. V wave onset detection in RVA: The V wave maximum in the RVA will be searched within the 50 ms around the mapping point from the R wave. Its onset is also backward corrected in a 60 ms window using the threshold Thr_V_RVA.

4. A, H wave detection: The placement of the stimulation pulse plays an important role for the A wave detection. According to the distance between the stimulation pulse and the current V wave onset, two sub-branches are considered for the A, H detection. Sub-branch 1 is applied if the distance between the stimulation pulse and the current V wave onset is larger than 200 ms. The same H wave detection as in branch 1 is also used here. Sub-branch 2 is applied if the distance between the stimulation pulse and the current V wave onset is smaller than 200 ms.

Branch 3

The difference between the branch 3 and branch 2 is that two antegrade stimulation pulses can occur in the R-R interval, e.g. during the fast continuous pacing or gradual decrease of the pacing interval (e.g., Wenckbach point analysis). In many cases, the second antegrade pacing leads to the coincidence of the atrial and ventricular excitation, which makes it difficult to separate the A, H and V waves. Therefore, the time window for searching the A wave after the first pacing is defined as [Stimulation pulse1 + 20ms: Stimulation pulse 2 - 20ms], while the end of the window should not be closer than 150 ms to the V wave onset. The maximum in this time window that is over the threshold is detected as A wave in the HRA or CS signal. Then, the corresponding A wave will be further detected in the HIS signal. The beginning of the window in the HIS signal is the middle position between the first stimulation pulse and the A_HRA/CS; the end of the window is 50ms after the A_HRA/CS but 80ms before the V_HIS.

Branch 4

In this branch, a retrograde stimulation pulse is placed in the R-R interval in the RVA signal. The retrograde pacing causes inversed cardiac conduction from ventricular to atrial. That is, the wave sequence after the stimulation pulse is first a V wave and then an A wave in the normal case. The V wave and A wave can often coincide, which enhances the difficulty to detect the A wave in the HIS signal.

1. V wave detection in HIS and HRA/CS: The position of the Q wave is considered as that of the V wave.

2. V wave detection in RVA: Because the pacing is placed in the RVA signal, the V wave will not be earlier than 10 ms after the stimulation pulse.

3. A wave detection in HRA/CS: Due to the retrograde conduction, the A wave in the current R-R interval is induced by the retrograde stimulation pulse in the last R-R interval. The window of searching the A wave in HRA or CS signal begins from 120 ms after the first V wave in the current R-R interval. The end of the window is 50 ms before the stimulation pulse in the current R-R interval. If the maximum in this window is over the threshold Thr_A_HRA/CS, then it is considered as A wave and its onset is backward corrected in a 30 ms window.

4. A wave detection in HIS: The window [A_HRA/CS - 30ms: A_HRA/CS + 50ms] is applied to further search the maximum in the HIS signal. The maximum is detected as the A wave in the HIS signal. Its onset is also backward corrected in a 30 ms window.

5. Spontaneous A, H, V wave detection after the last retrograde stimulation pulse. It is mentioned that more attention should be paid to the last retrograde stimulation pulse. After the retrograde conduction induced by the last stimulation pulse, a spontaneous wave sequence can be followed. The identification of the spontaneous waves is the same as that explained in the branch 1.

Branch 5

Comparing to branch 1, only the HIS signal is acquired for the wave identification. No stimulation pulse is placed in the R-R interval. It is not easy to separate the A and H wave in the HIS signal without any other reference signal. As a solution, the detection of the P wave in the body surface ECG signal II is used as the reference to define the window of the A wave detection.

1. V wave onset detection in HIS: The same procedure is applied as in branch 1.

2. A wave detection in the HIS: The window [P onset: P onset + 60ms] is applied to search the maximum in the HIS signal. The maximum is detected as the A wave in the HIS signal. The onset correction of the A wave consists of two steps: first, it is backward corrected by the threshold Thr_A_HIS in a 30 ms window in the HIS signal; second, a forward correction is done on the filtered HIS signal.

3. H wave detection in the HIS: The same procedure is applied as in branch 1.

**Claims**

1.  A method for analysing an intracardiac electrocardiogram (IECG) consisting of one or several electrogram signals each acquired by a catheter (HRA, RVA, CS, HIS) placed inside the heart, in order to identify at least one of the A wave, V wave and H wave on at least one of the electrogram signals, wherein the method also uses a simultaneously acquired body surface electrocardiogram (BSECG), comprising the following steps:

    - pre-processing (14) the electrogram signal;
    - identifying (12) the QRS-complex in the body surface electrocardiogram;
    - calculating an adaptive threshold for the A, V or H wave, wherein the adaptive threshold depends on the noise level of the electrogram signal and on the type of wave;
    - identifying (12) the A, V or H wave by searching the electrogram signal within a time window determined either from the position of the QRS complex in the BSECG, or the previously determined position a wave on the same or another electrogram signal, and
    - detecting the A, V or H wave within the time window using the adaptive threshold.

2.  A method of analysing an intracardiac electrocardiogram (IECG) consisting of one or several electrogram signals each acquired by a catheter (HRA, RVA, CS, HIS) placed inside the heart, in order to identify at least two of the A wave, V wave and H wave on at least one of the electrogram signals, in particular according to claim 1, wherein the method also uses a simultaneously acquired body surface electrocardiogram (BSECG), comprising the following steps:

    - pre-processing (14) the electrogram signal;

- identifying (12) the QRS-complex in the body surface electrocardiogram;
- defining an R-R interval between two subsequent R waves on the BSECG;
- checking (16) the R-R interval for the presence of stimulation pulses, and
- selecting (18) one of several procedural branches (20) for identifying the A wave, H wave and V wave, the selection depending on the position of the catheter(s), the number of stimulation pulses and the type of stimulation.

3. The method of claim 1 or 2, wherein the pre-processing comprises

- applying a high-pass or band stop filter (26) on the electrogram signal;
- applying a nonlinear transformation (30) on the filtered signal, in particular a Hilbert transform, in order to extract an envelope of the filtered signal;
- applying a low-pass filter (32) on the envelope of the filtered signal.

4. The method of one of the preceding claims, wherein the pre-processing (14) comprises removing possible pacing artefacts (14) resulting from stimulation pulses from the electrogram signal by means of the following steps:

- detecting the ascending or descending edge of a stimulation wave on a stimulation marker signal, which is supplied by the electrophysiological study system generating the stimulation pulses;
- setting the electrogram signal to zero within a pre-determined time window around the detected ascending or descending edge.

5. The method of one of the preceding claims, wherein the electrogram signal is the HIS signal, i.e. it is acquired by a catheter (HIS) placed near the bundles of His.

6. The method of claim 5, wherein at least one or several further electrogram signals are acquired by a catheter or catheters (HRA, CS, RVA) placed in the right atrium (the HRA signal), in the coronary sinus (the CS signal), or near the right ventricular apex (the RVA signal).

7. The method of claim 6, wherein a first procedural branch (20) for identifying the A wave, H wave and V wave in the HIS signal and the A wave and V wave on the HRA or CS signal comprises the following steps:

- defining an R-R interval between two subsequent R waves on the BSECG;
- where necessary, calculating an adaptive threshold for each of the V wave, A wave and H wave in each of the HIS, HRA/CS and RVA signals, wherein the adaptive threshold depends on the type of wave and on the noise level of the respective signal in the R-R interval;
- identifying the V wave in the HIS signal by searching the signal within a first time window determined from the position of the QRS complex, and using the respective adaptive threshold to detect the V wave;
- identifying the V wave in the HRA or CS signal by searching the signal within a second time window determined from the detected onset of the V wave in the HIS signal, and using the respective adaptive threshold to detect the V wave;
- identifying the A wave in the HRA or CS signal by searching the signal within a third time window determined from the detected onset of the V wave in the HIS signal, and using the respective adaptive threshold to detect the A wave;
- if an A wave is detected on the HRA or CS signal, identifying the A wave in the HIS signal by searching the signal within a fourth time window determined from the detected onset of the A wave in the HRA or CS signal, and using the respective adaptive threshold to detect the A wave;
- identifying the H wave in the HIS signal by searching the signal within a fifth time window determined from the detected positions of the A wave and the V wave and their distance in the HIS signal, and using the respective adaptive threshold to detect the H wave.

8. The method of claim 7, wherein the adaptive thresholds are calculated after pre-processing (14) of the signals, and additional threshold values are calculated from the signals before pre-processing.

9. The method of claim 7 or 8, wherein the first procedural branch (20) is used when no stimulation pulses are detected in the R-R interval, and other branches, which are used for electrogram signals acquired during antegrade stimulation, are variations of the first branch.

10. An electrophysiological system comprising

- catheters (HRA, RVA, CS, HIS) for acquiring an intracardiac electrocardiogram (IECG) when inserted into the heart,
- leads for acquiring a body surface electrocardiogram (BSECG),
- a signal processor for amplifying and filtering the acquired signals, and
- a data analysis station, which is adapted for performing the method according to one of the preceding claims.

11. A computer program product containing program code which will allow a computer on which the program is installed to perform the method according to one of claims 1 to 10.

## FIG 1

## FIG 2

FIG 3

## FIG 4

| 24 | | 26 | | 28 | | 30 | | 32 |
|---|---|---|---|---|---|---|---|---|
| Pacing artifact removal | → | Band Stop filter | → | Absolute value | → | Non-linear transform | → | Low-Pass filter | →

## FIG 5

30

$()^2$

Hilbert transform    $()^2$

34

$+$    $\sqrt{\phantom{x}}$

## FIG 6

A    raw signal

B    after step 26

C    after step 30

D    after step 32

# FIG 7

```
┌─────────────────────────────┐
│  V wave onset detection on  │
│             HIS             │
└─────────────────────────────┘
              │
              ▼
┌─────────────────────────────┐
│  V wave onset detection on  │
│           HRA/CS            │
└─────────────────────────────┘
              │
              ▼
┌─────────────────────────────┐
│  V wave onset detection on  │
│             RVA             │
└─────────────────────────────┘
              │
              ▼
┌─────────────────────────────┐
│     A wave detection and    │
│  onset correction on HRA/CS │
└─────────────────────────────┘
              │
              ▼
┌─────────────────────────────┐
│     A wave detection and    │
│    onset correction on HIS  │
└─────────────────────────────┘
              │
              ▼
┌─────────────────────────────┐
│   H wave detection on HIS   │
└─────────────────────────────┘
```

FIG 8

**European Patent Office**

## PARTIAL EUROPEAN SEARCH REPORT

which under Rule 45 of the European Patent Convention shall be considered, for the purposes of subsequent proceedings, as the European search report

**Application Number**

EP 06 00 0737

### DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | EP 1 190 671 A (BIOSENSE, INC) 27 March 2002 (2002-03-27) * the whole document * ----- | 10 | A61B5/0452 G06F17/00 G06F19/00 |
| A | US 2004/059237 A1 (NARAYAN SANJIV MATHUR ET AL) 25 March 2004 (2004-03-25) * the whole document * ----- | 10 | |
| A | US 6 064 906 A (LANGBERG ET AL) 16 May 2000 (2000-05-16) * the whole document * ----- | 10 | |
| A | US 5 743 859 A (WODLINGER ET AL) 28 April 1998 (1998-04-28) * the whole document * ----- | 10 | |

**TECHNICAL FIELDS SEARCHED (IPC)**

A61B
G06F

### INCOMPLETE SEARCH

The Search Division considers that the present application, or one or more of its claims, does/do not comply with the EPC to such an extent that a meaningful search into the state of the art cannot be carried out, or can only be carried out partially, for these claims.

Claims searched completely :

Claims searched incompletely :

Claims not searched :

Reason for the limitation of the search:

see sheet C

| Place of search | Date of completion of the search | Examiner |
|---|---|---|
| Munich | 8 March 2006 | Sopelana Martínez, J |

European Patent
Office

**INCOMPLETE SEARCH
SHEET C**

Application Number

EP 06 00 0737

Claim(s) searched completely:
    10

Claim(s) not searched:
    1-9,11

Reason for the limitation of the search (non-patentable invention(s)):

Article 52 (4) EPC - Method for treatment of the human or animal body by surgery
Article 52 (2)(c) EPC - Program for computers

## ANNEX TO THE EUROPEAN SEARCH REPORT
## ON EUROPEAN PATENT APPLICATION NO.

EP 06 00 0737

This annex lists the patent family members relating to the patent documents cited in the above-mentioned European search report.
The members are as contained in the European Patent Office EDP file on
The European Patent Office is in no way liable for these particulars which are merely given for the purpose of information.

08-03-2006

| Patent document cited in search report | | Publication date | Patent family member(s) | | Publication date |
|---|---|---|---|---|---|
| EP 1190671 | A | 27-03-2002 | AU | 781902 B2 | 23-06-2005 |
| | | | AU | 7607501 A | 28-03-2002 |
| | | | CA | 2357729 A1 | 22-03-2002 |
| | | | JP | 2002172097 A | 18-06-2002 |
| | | | US | 2002151808 A1 | 17-10-2002 |
| US 2004059237 | A1 | 25-03-2004 | AU | 2003299035 A1 | 08-04-2004 |
| | | | WO | 2004026123 A2 | 01-04-2004 |
| US 6064906 | A | 16-05-2000 | AU | 6551498 A | 12-10-1998 |
| | | | WO | 9841144 A1 | 24-09-1998 |
| | | | US | 5772604 A | 30-06-1998 |
| US 5743859 | A | 28-04-1998 | NONE | | |

EPO FORM P0459

For more details about this annex : see Official Journal of the European Patent Office, No. 12/82